# NEUE EUROPÄISCHE PATENTSCHRIFT

(11) **EP 2 108 328 B2**
(45) Veröffentlichungstag und Bekanntmachung des Hinweises auf die Entscheidung über den Einspruch: **26.08.2020**
(45) Hinweis auf die Patenterteilung: 29.02.2012
(21) Anmeldenummer: 08154245.8
(22) Anmeldetag: 09.04.2008
(51) Int. Cl.: A61B 19/00

(54) **Bildbasiertes Ansteuerungsverfahren für medizintechnische Geräte**
Image-based control method for medicinal devices
Procédé de commande basée sur l'image pour appareils médicaux

(43) Veröffentlichungstag der Anmeldung: 14.10.2009
(73) Patentinhaber: Brainlab AG, 81829 München (DE)
(72) Erfinder: Steinle, Wolfgang, 80337 München (DE); Frielinghaus, Nils, 85551 Heimstetten (DE); Hamilton, Christoffer, 81371 München (DE)
(74) Vertreter: SSM Sandmair

(56) Entgegenhaltungen:
- WO-A-2007/017642
- WO-A1-2006/040697
- US-A- 5 099 846
- US-A1- 2005 228 256
- US-A1- 2008 072 151
- US-B1- 6 201 984
- US-B2- 6 638 223
- Rekimoto, J.: "SmartSkin: an infrastructure for freehand manipulation on interactive surfaces", CHI 2002 Conference Proceedings Conference on Human Factors in Computing Systems, 20 April 2002 (2002-04-20), - 25 April 2002 (2002-04-25), pages 113-120,

## Beschreibung

Die Erfindung betrifft ein bildbasiertes Ansteuerungsverfahren für medizinische Geräte. Insbesondere werden im Rahmen der Erfindung bilderzeugende medizinische Geräte angesteuert, wie z.B. Endoskope, CT-, MR-, Ultraschall-, oder Videogeräte.

Derzeit werden medizintechnische bilderzeugende Geräte mit einer Reihe von Eingabegeräten versehen, wie z.B. Knöpfen, Joysticks, Schaltern und Software-Benutzerschnittstellenelementen, und Parameter für die Bilderzeugungsgeräte werden softwareseitig mit Benutzerschnittstellen eingegeben wie z.B. Texteingabefeldern, Scroll-Balken und ähnlichem.

Andere Geräte werden durch Bediener-Interaktion gesteuert, wie z.B. in den Fällen, wo ein Behandelnder einen Ultraschallkopf hält und ihn an verschiedenen Punkten eines Patientenkörpers positioniert. Aus der US 5,558,619 ist ein Endoskopsystem bekannt, das eine automatische Steuerung in Abhängigkeit von der Bewegung eines Bedieners umfasst. Aus der US 6,778,846 B1 sind ein Verfahren und ein System zum Führen eines medizinischen Geräts innerhalb eines Patienten unter Verwendung von Bilddaten bekannt.

Aus der US 2004/0030219 A1 ist ein Endoskop mit einer Kamera bekannt, wobei die Kamera mittels eines Touchscreens angesteuert werden kann.

Es ist die Aufgabe der vorliegenden Erfindung, die Steuerung medizintechnischer Geräte, insbesondere medizintechnischer bildgebender Geräte zu optimieren. Insbesondere soll die Steuerung direkter gemacht und besser auf den Bedarf des Verwenders abgestimmt werden.

Diese Aufgabe wird erfindungsgemäß durch ein Verfahren gemäß dem Patentanspruch 1 gelöst. Die Unteransprüche definieren bevorzugte Ausführungsformen der Erfindung.

Beim erfindungsgemäßen Verfahren werden Patientenbilddaten durch ein bilderzeugendes medizintechnisches Gerät erzeugt und ein Patientenbild wird auf der Basis dieser Daten erstellt. Dieses Bild wird dann auf einer Bildsausgabeeinheit dargestellt. Dann wiederum findet eine Interaktion mit dem Bild statt, die ein medizintechnisches Gerät, z.B. das bilderzeugende Gerät oder ein anderes bilderzeugendes Gerät (die Bilderzeugung) ansteuert.

Mit anderen Worten schließt die Erfindung einen bisher offenen Kreis zwischen Bilderzeugung, Bilddarstellung und Bildbetrachtung, indem sie eine Interaktion mit dem Bild ermöglicht und das Resultat dieser Interaktion dann wieder zurück auf das bilderzeugende Gerät wirken lässt, also auf die Bildherstellung. Die Erfindung verwendet also die erzeugten Bilder, um wiederum die Bilderzeugung zu steuern bzw. zu kontrollieren. Sie ermöglicht es dadurch, auf Eingabegeräte (indirekte Eingabegeräte) wie Knöpfe, Joysticks, Schalter und Softwareschnittstellen zu verzichten und trotzdem gewünschte Änderungen in der Bildwiedergabe zu aktivieren. Der Bediener muss also nur das Bild manipulieren, um zum gewünschten neuen Bild zu gelangen. Vorteilhafterweise wird hierdurch ein direkterer Weg der Interaktion mit den medizintechnischen Geräten ermöglicht, und gewünschte Bilder oder Ansichten können in einfacher Weise generiert werden. Ferner besteht die Möglichkeit, dem Verwender einen einfachen und sehr intuitiven Zugang zu wichtigen Funktionen zu bieten, und es können beispielsweise Details der Bedienung medizinischer Vorrichtungen für den Bediener vollständig verborgen bleiben, was die Bilderzeugung stark vereinfacht.

Die Gerätesteuerung erfolgt durch eine Interaktion an der Bildausgabeeinheit, insbesondere durch Touch-Eingaben oder Multi-Touch-Eingaben. Eine weitere nicht erfindungsgemäße Möglichkeit besteht in der Blickerfassung und/oder Augenbewegungserfassung, wobei es möglich ist, die Bildausgabeeinheit ein- oder auszuschalten, je nach dem, ob ein Betrachter das Patientenbild anblickt oder nicht. Auch kann das Bild an der Stelle verändert werden, die gerade betrachtet wird (z.B. vergrößert, aufgehellt oder kontrastreicher gemacht werden).

Grundsätzlich kann durch die erfindungsgemäße Gerätesteuerung in verschiedener Weise auf die Bilddaten Einfluss genommen werden. Es können neue Bilddaten erzeugt werden, neue Bilddaten können aus einem Speicher abgerufen werden und/oder die Bilddaten können durch die Gerätesteuerung verändert werden. Die Neuerzeugung umfasst dabei oftmals eine Änderung der Bildaufnahmeparameter und eine neue Bildaufnahmeprozedur. Beim Abrufen neuer Daten können diese in Form eines Patientendatensatzes vor Ort oder auf einem Netzwerk zur Verfügung stehen, und die Änderung der Patientendaten kann beispielsweise als Reaktion auf Eingaben eines Arztes erfolgen, der über die Bildausgabe Planungseingaben macht (Markierungen, Operationsanweisungen etc.).

Es besteht im Rahmen der Erfindung die Möglichkeit, durch die Gerätesteuerung am bilderzeugenden Gerät einen Bildaufnahmeparameter verändern, wobei insbesondere eine oder mehrere der folgenden Manipulationen vorgenommen wird bzw. werden:
- Änderung der Position des Geräts oder seines Bilderfassungselements;
- Änderung der Bilderfassungsauflösung;
- Änderung des Bilderfassungswinkels;
- Änderung oder Verschiebung des Bilderfassungsbereiches;
- Änderung von Kontrast- oder Helligkeitseinstellungen;
- Änderung der Eindringtiefe;
- Änderung der Schichterfassungstiefe.

Es sind mehrere konkrete Ausführungsformen des erfindungsgemäßen Verfahrens denkbar, bei denen eine oder mehrere der folgenden Tätigkeiten im Rahmen der bildbasierten Gerätesteuerung durchgeführt werden wird bzw. werden:
- die Bildaufnahmeeigenschaften eines Endoskops werden verändert,
- dem Patientenbild werden aus Bildgebungsdatensätzen (CT, MR) erhaltene, rekonstruierte Bilder hinzugefügt, überlagert oder das Patientenbild wird damit zumindest teilweise ersetzt;
- die Bildaufnahmeparameter eines medizinischen Bildgebungsgeräts (CT, MR, PET, etc.) werden verändert und es wird eine neue Bilderfassung mit den geänderten Parametern durchgeführt;
- die Schalleindringparameter einer Ultraschallsonde oder deren Position werden verändert;
- die Bildaufnahmeeigenschaften eines Mikroskops werden verändert;
- bildverbessernde Substanzen, insbesondere Kontrastmittel, werden dem Patienten verabreicht;
- EKG- oder Herzschrittmacher-Parameter werden verändert;
- die Raumbeleuchtung oder das Operationslicht werden verändert, insbesondere auf einen Ort konzentriert, der auf dem Patientenbild angezeigt wird;
- ein im Bild erscheinendes, automatisch manipulierbares Instrument wird durch Manipulation, insbesondere Verschiebung, seines Bildes entsprechend realiter manipuliert, insbesondere verschoben;
- die Strahlungsparameter und/oder Bildaufnahmeeigenschaften eines LINACs werden verändert;
- in das Bild eingezeichnete Bereiche werden lokalisiert und insbesondere dann auf den Patienten projiziert.

Wiederum sehr allgemein gesprochen, kann das erfindungsgemäße Verfahren so durchgeführt werden, dass die neuen oder veränderten Bilddaten dazu verwendet werden, das Patientenbild auf der Bildausgabeeinheit zu erneuern, zu verändern oder zu ergänzen.

Die Erfindung betrifft ferner ein Programm, das, wenn es auf einem Computer läuft oder in einem Computer geladen ist, den Computer veranlasst, ein Verfahren durchzuführen, wie es oben in verschiedenen Ausführungsformen beschrieben wurde. Ferner betrifft sie ein Computerprogramm-Speichermedium, das ein solches Programm aufweist.

Die Erfindung wird im Weiteren anhand mehrerer Ausführungsformen detaillierter beschrieben. Sie kann alle hierin beschriebenen Merkmale einzeln sowie in jedweder sinnvollen Kombination umfassen und auch als Vorrichtungs-Erfindung aufgefasst werden, wenn die beschriebenen oder notwendigen Geräte derart zusammengestellt werden, dass ein hierin beschriebenes Verfahren umgesetzt wird. Die einzige beiliegende Figur zeigt schematisch einen erfindungsgemäßen Steuerungskreis anhand einer Zoom-Verstellung.

In der beiliegenden Figur ist schematisch eine Bildanzeigeeinheit 1 dargestellt, die im vorliegenden Falle ein Touch-Screen-Monitor ist. Ferner ist rechts daneben in der Figur ein wiederum nur schematisch dargestelltes bilderzeugendes Gerät 4 gezeigt. Die Bezugszeichen 2 und 3 zeigen schematisch Bilder vor und nach einer Änderung, und die Buchstaben A, B, C und D veranschaulichen die Schritte des Verfahrensablaufes.

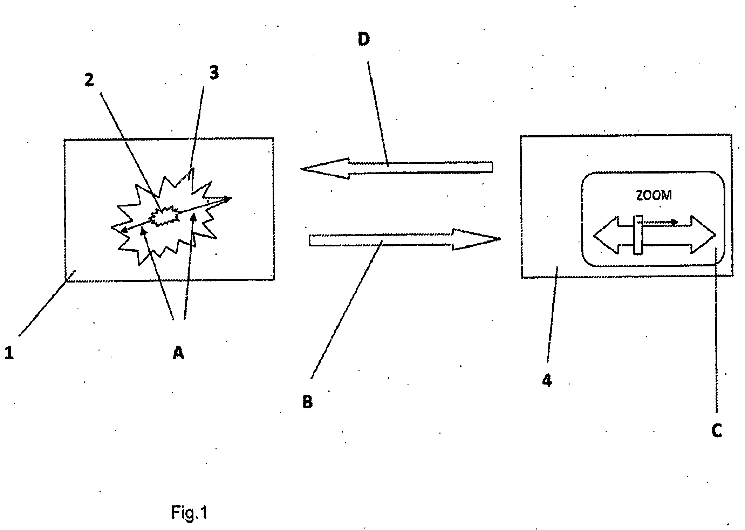

Ein allgemeiner Verfahrensablauf gemäß der vorliegenden Erfindung wäre nun der folgende: Die Bildausgabe 1 zeigt zunächst nur das Bild 2, beispielsweise ein Patientenkörperteil, welches mit Hilfe des Bildaufnahmegerätes 4 aufgenommen wird oder wurde; die Bildaufnahmedarstellung kann auf der Basis neuer oder gespeicherter Bilder erfolgen.

Wenn nun ein Bediener das Objekt 2 in höherer Auflösung oder größer betrachten möchte, geht er zum Bildschirm 1, der eine Multitouch-Oberfläche aufweist. Er "greift" das Bild 2 an zwei Stellen und zieht es dann gemäß den Pfeilen A auseinander, so dass es die Größe des Bildes 3 erreicht.

Diese Eingabe am Bildschirm 1 wird als Gerätesteuerungsinformation an das bilderzeugende Gerät 4 weitergeleitet, was durch den Pfeil B dargestellt ist. Dabei kann die Zoom-Bewegung (A) schon am Bildschirm durch eine Grafikverarbeitungseinheit oder auch erst später am medizinischen Bildaufnahmegerät in einen Gerätesteuerungsbefehl umgesetzt werden.

Im vorliegenden Fall ist gezeigt, dass die Information B über das Aufzoomen beim Bilderzeugungsgerät 4 ankommt, wo sie in einen Zoombefehl umgewandelt wird, der durch den Kasten C schematisch dargestellt ist. Dieser Zoombefehl bewirkt nun eine Änderung in den Bildaufnahmeparametern des Geräts 4. Beispielsweise wird das Aufnahmeelement (Bilderfassungselement wie Kamera, Linse, etc.) näher an das Objekt herangefahren oder die Auflösung wird vergrößert.

Die mit den veränderten Aufnahmeparametem erzeugten neuen Bilddaten werden dann, wie mit dem Pfeil D dargestellt, wieder an den Monitor 1 gesendet, und an diesem wird das Bild 3 nunmehr mit der verbesserten Auflösung in der größeren Gestalt dargestellt. Damit schließt sich der Kontroll- bzw. Steuerungskreis und der Bediener hat ein neues Bild zur Verfügung, ohne am Gerät 4 selbst manipulieren zu müssen.

Verschiedene Ausführungsformen werden im Weiteren nun detaillierter erörtert:

Endoskop: Das bilderzeugende, medizintechnische Gerät kann ein Endoskop sein. Ein Endoskop hat im Allgemeinen eine starre oder flexible Röhre, die beim Einbogen in einen Patienten ein Bild zurück (durch die Röhre) liefert. Es werden Gesten, die an dem Bild durchgeführt werden, dazu verwendet, die Position und/oder andere Parameter des Endoskops zu modifizieren. Solche Gesten können hier und ganz allgemein Gesten sein, die mit Berührung an einem berührungsempfindlichen Monitor durchgeführt werden (Touchscreen) oder auch solche, die durch Gestenerkennungssysteme identifiziert werden und nicht unbedingt eine Berührung eines Monitors oder Bildausgabegerätes erfordern. Beispielweise kann im vorliegenden Fall die Gestenerkennung dazu führen, dass die Spitze der Endoskopröhre nach vorne (Einzoomen) oder nach hinten (Auszoomen) bewegt wird. Eine solche Bewegung kann mechanisch unterstützt sein, wobei die Steuerungsbefehle dann auf diese Mechanik wirken. Auch die Endoskop-Blickrichtung kann verändert werden, indem beispielsweise eine Verschiebegeste am Bild ausgeführt wird, wodurch die Endoskopspitze in jedweder Richtung geschoben werden kann. Durch eine rotierende Steuerbewegung kann beispielsweise ein Rotieren des Endoskops ausgelöst werden. Andere Gesten könnten verwendet werden, um die Lichtintensität zu erhöhen oder zu verringern, die Linse des Endoskops zu wechseln oder jedwede andere Tätigkeit durchzuführen, welche mit der Endoskop-Hardware möglich ist.

Rekonstruiertes Bild (anstelle des Endoskopbildes oder zusätzlich zum Endoskopbild): Bei der Steuerung eines Endoskops (aber auch bei der Steuerung anderer bildgebender Geräte) kann die Bildgebung erweitert werden, indem das erzeugte Bild (Endoskopbild) durch ein Bild überlagert wird, welches aus Kernspin- oder Computertomographiedaten rekonstruiert wird. Dabei kann die Überlagerung fließend übergehend zu gewünschten Anteilen stattfinden, die vom Gerät (Endoskop) beobachtete Region kann aber auch vollständig durch eine MR- oder CT-Rekonstruktion ersetzt werden, wenn beispielsweise ein Endoskopbild nicht verfügbar ist oder das Endoskop körperlich nicht zu der Position gebracht werden kann, an der das Bild zu erstellen ist.

MR/CT: Hier wird ein Bild, das durch einen Verwender betrachtet werden soll, durch einen Schichtbild-Scan erzeugt, wie z.B. MR (Kernspintomographie), CT (Computertomographie) oder PET (Positronen-Emissions-Tomographie) oder jedwede andere Vorrichtung, die Mehrschicht-Scans zeugt. Gemäß der Erfindung modifizieren hier die am Bild ausgeführten Gesten die Scan-Eigenschaften des Bilderzeugungsgeräts. Beispielsweise kann der Verwender einen interessierenden Bereich im Bild definieren, und das bilderzeugende Gerät führt dann einen detaillierteren neuerlichen Scan des interessierenden Bereiches durch. Eine Geste kann beispielsweise verwendet werden, um den Schichtbild-Erfassungsabstand bei einem solchen neuerlichen Scanvorgang zu verändern und dabei die Genauigkeit zu erhöhen. In einem anderen denkbaren Anwendungsfall bewirkt eine Verwendernavigationseingabe, welche die momentanen Grenzen des Datensatzes überschreitet bzw. überbeansprucht eine Neuakquirierung von Bildern. Dabei kann es von Vorteil sein, wenn der Patient bei der Auswertung der ersten Bilder auf dem Scanner-Tisch verbleibt.

Ultraschall: Zur Steuerung der Ultraschallbild-Akquirierung können Gesten des Verwenders auf einem Ultraschallbild beispielsweise die Eindringtiefe des Bildes verändern (Zoom-Bild). Bei einer TEE-Ultraschallerfassung (Transesofageal-Echokardiogramm) wird die Ultraschallsonde in der Speiseröhre des Patienten angeordnet und dreht sich, um mehrere Schichtaufnahmen zu akquirieren. Bei dieser Aufnahme kann beispielsweise eine Scrollgeste auf den Bildern verwendet werden, um die Sondendrehung zu verändern und dadurch unterschiedliche Schichten darzustellen. Eine Verschiebe-Geste auf den Bildern wird beispielsweise verwendet, um die Ultraschallsonde in der Speiseröhre nach oben oder nach unten zu bewegen.

Mikroskop: Bei der Verwendung eines medizintechnischen Mikroskops (pathologisches Mikroskop) können jedwede Zoom- oder Verschiebegesten auf den Bildern in Bewegungen der Mikroskoplinsen und/oder der Position des Mikroskops umgewandelt werden. Dies erzeugt für den Verwender den Eindruck, dass er durch einen Bildersatz hindurch "navigiert".

Akquirierungsparameter: Während beim Stand der Technik der Verwender die Helligkeit und den Kontrast von Bildern erst nach deren Akquirierung ändern kann, können. Verwendergesten auf dem Bild die Helligkeit und den Kontrast in dem Bereich verändern, der durch das Bild geliefert wird, aber auch in einem Bereich, den die Akquirierungsparameter des Geräts beeinflussen. Dies macht es möglich, mit dem Gerät die Bildakquirierung für exakt die Einstellungen zu verbessern, die der Verwender gewählt hat. In einer Erweiterung des Gedankens können Gesten definiert werden, welche wiederum jedwede Akquirierungsparameter des Geräts ändern können.

EKG: EKG-Kurven zeigen elektrische Herzaktivitäten. Interaktive Bilder, die eine EKG-Spur darstellen, können manipuliert werden. Modifikationen an der EKG-Kurve, z.B. das Beschleunigen oder Verlangsamen des Herzschlages, werden durch eine andere Vorrichtung interpretiert, welche Maßnahmen ergreift, um das derzeitige EKG auf das gewünschte zu ändern (z.B. Verabreichung von Dobutamin zur Beschleunigung des Herzschlages).

Schrittmacher: In Erweiterung des Gedankens der Herzschlagregelung ist es möglich, eine EKG-Kurve zu verwenden, um die Parameter eines Schrittmachers oder eines implantierbaren Kardioverter-Defibrillators zu modifizieren. Diese Vorrichtungen werden für jeden Patienten genau eingestellt, und durch das Ändern der Geräteparameter wird die Herzschlagrate geändert. Der Verwender kann gemäß der vorliegenden Erfindung direkt die EKG-Kurve (EKG-Diagramm) ändern, und diese Modifikation wird dann in geänderte Parameter für den Schrittmacher oder den Defibrillator umgesetzt. Dadurch können solche Geräte schneller und in einfacherer Weise feinabgestimmt werden. Hier zeigt sich auch, dass die dargestellten Bilddaten nicht unbedingt Bilder von tatsächlichen Objekten sein müssen, sondern auch Abbildungen wie Diagramme sein können, welche körperliche Funktionen wiedergeben. Auch diese Abbildungen werden im vorliegenden Fall als "Patientenbilder" bezeichnet.

Operationsraumbefeuchtung: Bei dieser Ausführungsform kann eine Bilddarstellungsvorrichtung verwendet werden, um ein anderes Gerät, das nicht unbedingt selbst ein Bild erzeugt aber die Bilderzeugung unterstützt, zu steuern. Indem ein Punkt auf einem Bild an der Bildausgabeeinheit ausgewählt wird, werden die Lichter im Operationsraum automatisch in eine Position gedreht, in der sie ihr Licht auf den gewählten Punkt konzentrieren. Dies geschieht vorteilhafterweise nach einer Registrierung des Patienten und der akquirierten Bilder in einem Navigations-/Trackingsystem. Wenn gemäß dieser Ausführungsform die beleuchtete Stelle heller wird, kann möglicherweise ein genaueres oder besseres Videobild gemacht werden, das dann alleine oder zusätzlich zum vorherigen Bild ebenfalls auf der Bildausgabeeinheit dargestellt werden kann. Es kann also das bildgebende Gerät, das durch die Manipulation angesteuert wird, dasselbe bildgebende Gerät sein, das das erste Bild gemacht hat (das manipulierte Bild) oder auch ein anderes bildgebendes Gerät, das zusätzliche Bilddaten erzeugt.

Steuerung eines Gerätes im Bild: Bei dieser Ausführungsvariante wird ein Bild verwendet, um eine Vorrichtung zu steuern, die auf dem Bild dargestellt wird. Ein Beispiel ist der Fall, wo ein intraoperatives Bild die Position einer Biopsienadel zeigt. Durch das Bewegen der Nadel im Bild (durch Eingabegesten) wird das tatsächliche körperliche Objekt automatisch bewegt, was eine exakte Positionierung ermöglicht.

LINAC: Zur Steuerung eines Linearbeschleunigers (Radiotherapie/Strahlentherapie) verwendet diese Variation Gesten, die das Bild manipulieren, um Strahlen bzw. Strahlenformen auszuwählen und zu verändern. Zusammen mit einem während der Prozedur erstellten Bild wird diese benutzt, um die Strahlen zu korrigieren und zu verschieben (Interaktion mit intra-prozeduralen Bildern).

Regionen oder Bilder, die am Patienten markiert werden: Hierbei kann jedwede Region (2- oder 3-dimensional) in ein medizinisches Bild eingezeichnet werden. Wenn die Patientenposition gegenüber den Bilddaten registriert ist, kann dann z.B. die exakte Lage der in der Bildausgabe eingezeichneten Region auf den Patienten projiziert werden, beispielsweise mit einem Laser.

Modifikation von Zubehör/anderen Geräten: Gemäß dieser Variation werden nichtmedizinische Bilder ausgegeben und Objekte in ihnen manipuliert. Ein Beispiel ist die Darstellung eines Bildes eines, Operationssaals, wobei durch die Manipulation des Bildes mit Hilfe von Gesten eine Anderung beispielsweise der Position oder Neigung eines Tisches ermöglicht wird. Ein weiteres Beispiel ist eine Dashboard-Software zur Operationsraum-Planung, bei der die Softwarebilder alle Operationsräume und ihren momentanen Status zeigen. Mit Gesten an den Türen und Aufzügen ist es dann möglich, deren Position zu ändern. Ein Beispiel ist das Öffnen aller Türen zu den Räumen, die von einem Gang her betretbar sind.

## Patentansprüche

1. Verfahren zur computergestützten Ansteuerung eines bilderzeugenden medizinischen Bildgebungsgeräts (4),
bei dem ein Schichtbild-Scan eines Patienten mit Bildaufnahmeparametern mittels des medizinischen Bildgebungsgeräts (4) erzeugt wird,
wobei ein Patientenbild (2) auf der Basis dieser Daten erstellt und auf einer Bildausgabeeinheit (1) dargestellt wird,
und wobei durch eine Berührung des Bildes (2) an der Bildausgabeeinheit (1) das medizinische Bildgebungsgerät (4) derart angesteuert wird,
dass die Bildaufnahmeparameter des medizinischen Bildgebungsgeräts (4) verändert werden und
ein neuer Schichtbild-Scan des Patienten mit den geänderten Parametern erzeugt wird,
wobei die Gerätesteuerung durch Multi-Touch-Eingaben erfolgt.

2. Verfahren nach Anspruch 1, bei dem die Gerätesteuerung durch Manipulation des Bildes erfolgt, insbesondere durch Vergrößern, Verkleinern oder Aufsichtsebenenverschiebung.

3. Verfahren nach einem der Ansprüche 1 bis 2, bei dem durch die Gerätesteuerung neue Bilddaten aus einem Speicher abgerufen werden.

4. Verfahren nach einem der Ansprüche 1 bis 3, bei dem durch die Gerätesteuerung Bilddaten verändert werden.

5. Verfahren nach einem der Ansprüche 1 bis 4, bei dem durch die Gerätesteuerung am bilderzeugenden Gerät ein Bildaufnahmeparameter verändert wird, insbesondere eine oder mehrere der folgenden Manipulationen vorgenommen wird bzw. werden:
- Änderung der Position des Geräts oder seines Bilderfassungselements;
- Änderung der Bilderfassungsauflösung;
- Änderung des Bilderfassungswinkels;
- Änderung oder Verschiebung des Bilderfassungsbereiches;
- Änderung von Kontrast- oder Helligkeitseinstellungen;
- Änderung der Eindringtiefe;
- Änderung der Schichterfassungstiefe.

6. Verfahren nach einem der Ansprüche 1 bis 5, bei dem eine oder mehrere der folgenden Tätigkeiten im Rahmen der bildbasierten Gerätesteuerung durchgeführt wird bzw. werden:
- dem Patientenbild werden aus Bildgebungsdatensätzen (CT, MR) erhaltene, rekonstruierte Bilder hinzugefügt, überlagert oder das Patientenbild wird damit zumindest teilweise ersetzt;
- die Schalleindringparameter einer Ultraschallsonde oder deren Position werden verändert.

7. Verfahren nach einem der Ansprüche 1 bis 6, bei dem die neuen oder veränderten Bilddaten verwendet werden, um das Patientenbild (2) auf der Bildausgabeeinheit (1) zu erneuern, zu verändern oder zu ergänzen.

8. Programm, das, wenn es auf einem Computer läuft oder in einem Computer geladen ist, den Computer veranlasst, ein Verfahren gemäß einem der Ansprüche 1 bis 7 durchzuführen.

9. Computerprogramm-Speichermedium, das ein Programm nach Anspruch 8 aufweist.

## Claims

1. A method for the computer-assisted control of an image-generating medical imaging apparatus (4), wherein: a tomographic scan of a patient is generated by means of the medical imaging apparatus (4) using image recording parameters, wherein a patient image (2) is produced on the basis of these data and displayed on an image output unit (1); and the medical imaging apparatus (4) is controlled by touching the image (2) on the image output unit (1), such that the image recording parameters of the medical imaging apparatus (4) are altered and a new tomographic scan of the patient is generated using the changed parameters, wherein the apparatus is controlled using multi-touch inputs.

2. The method according to claim 1, wherein the apparatus is controlled by manipulating the image, in particular by increasing/decreasing it in size, rotating it or shifting the viewing plane.

3. The method according to any one of claims 1 to 2, wherein new image data are retrieved from a memory using the apparatus control.

4. The method according to any one of claims 1 to 3, wherein image data are altered using the apparatus control.

5. The method according to any one of claims 1 to 4, wherein an image recording parameter is altered at the image-generating apparatus using the apparatus control and in particular one or more of the following manipulations is/are performed:
- changing the position of the apparatus or of its image detection element;
- changing the image detection resolution;
- changing the image detection angle;
- changing or shifting the image detection range;
- changing contrast or brightness settings;
- changing the penetration depth;
- changing the layer detection depth.

6. The method according to any one of claims 1 to 5, wherein one or more of the following actions is/are performed within the framework of image-based apparatus control:
- reconstructed images obtained from imaging data sets (CT, MR) are added to or superimposed onto the patient image, or the patient image is at least partially replaced with them;
- the sound penetration parameters or position of an ultrasound probe are altered.

7. The method according to any one of claims 1 to 6, wherein the new or altered image data are used to renew, alter or supplement the patient image (2) on the image output unit (1).

8. A program which, when it is running on a computer or is loaded onto a computer, causes the computer to perform a method in accordance with any one of claims 1 to 7.

9. A computer program storage medium which comprises a program according to claim 8.

## Revendications

1. Procédé de commande par ordinateur d'un appareil d'imagerie médicale (4) qui généré des images, dans lequel un balayage d'image en couches d'un patient est généré en utilisant des paramètres de capture d'image au moyen de l'appareil d'imagerie médicale (4), dans lequel une image de patient (2) est généré sur la base de ces données et affichée sur une unité de sortie d'image (1), et dans lequel l'appareil d'imagerie médicale (4) est commandé par un contact avec l'image (2) de telle sorte que les paramètres de capture d'image de l'appareil d'imagerie médicale (4) sont modifiés et un nouveau balayage d'image en couches du patient est généré en utilisant les paramètres modifies, dans lequel les appareils sont commandés par des entrées tactiles multiples.

2. Procédé selon la revendication 1, dans lequel les appareils sont commandés par une manipulation de l'image, en particulier par un agrandissement, une réduction, une rotation ou un déplacement de plan d'observation.

3. Procédé selon l'une quelconque des revendications 1 à 2, dans lequel de nouvelles données d'image sont récupérées à partir d'une mémoire en utilisant la commande d'appareil.

4. Procédé selon l'une quelconque des revendications 1 à 3, dans lequel des données d'image sont modifiées en utilisant la commande d'appareil.

5. Procédé selon l'une quelconque des revendications 1 à 4, dans lequel un paramètre de capture d'image est modifié en utilisant la commande d'appareil sur l'appareil générateur d'image, en particulier en exécutant une ou plusieurs des manipulations suivantes:
- changement de la position de l'appareil ou de son élément de détection d'image,
- changement de la résolution de détection d'image,
- changement de l'angle de détection d'image,
- changement ou déplacement de la région de détection d'image,
- changement des réglages de contraste ou de luminosité,
- changement de la profondeur de pénétration,
- changement de la profondeur de détection de couche.

6. Procédé selon l'une quelconque des revendications 1 à 5, dans lequel une ou plusieurs des actions suivantes sont exécutées dans le cadre de la commande d'appareil à base d'images:
- des images reconstruites, obtenues à partir d'ensembles de données d'imagerie (GT, MR) sont ajoutées ou superposées à l'image du patient, ou l'image du patient est au moins partiellement remplacée par celles-ci,
- les paramètres de pénétration du son d'une sonde à ultrasons ou sa position sont changes.

7. Procédé selon l'une quelconque des revendications 1 à 6, dans lequel les données d'image nouvelles ou modifiées sont utilisées afin de renouveler, de modifier au de compléter l'image du patient (2) sur l'unité de sortie d'image (1 ).

8. Programme qui, lorsqu'il s'exécute sur un ordinateur ou lorsqu'il est chargé dans un ordinateur, amène l'ordinateur à mettre en oeuvre un procédé selon l'une quelconque des revendications 1 à 7.

9. Support de mémoire de programme d'ordinateur comportant un programme selon la revendication 8.
